# EUROPEAN PATENT APPLICATION

(11) **EP 4 785 957 A1**
(43) Date of publication of application: **05.08.2026**
(21) Application number: 24870602.0
(22) Date of filing: 20.09.2024
(51) Int. Cl.: A61K 39/40, A61K 39/395, C07K 16/12, A61P 31/04, A61P 21/02

(54) **APPLICATION OF ANTI-TETANUS TOXIN ANTIBODY IN PREVENTING OR TREATING TETANUS**

(30) Priority: 27.09.2023 CN 202311265343
(71) Applicant: Zhuhai Trinomab Pharmaceutical Co., Ltd., Zhuhai, Guangdong 519090 (CN)
(72) Inventor: LIAO, Huaxin, Zhuhai, Guangdong 519090 (CN); ZHENG, Weihong, Zhuhai, Guangdong 519090 (CN); WANG, Yueming, Zhuhai, Guangdong 519090 (CN); WANG, Wanmei, Zhuhai, Guangdong 519090 (CN); KUANG, Xiaohu, Zhuhai, Guangdong 519090 (CN); DING, Jiyuan, Zhuhai, Guangdong 519090 (CN); LIU, Xinyu, Zhuhai, Guangdong 519090 (CN)
(74) Representative: Bianchetti & Minoja with Trevisan & Cuonzo IPS SRL
(86) International application number: PCT/CN2024/119974
(87) International publication number: WO 2025/067058

(57) **Abstract**

Provided is an application of an anti-tetanus toxin antibody in preventing or treating tetanus. The anti-tetanus toxin antibody is applied in a specific administration regimen, and can achieve the technical effects of being quicker to take effect and having a longer protection time than existing passive immunizing agents, and can therefore effectively prevent or treat tetanus.

## Description

### CROSS-REFERENCES TO RELATED APPLICATIONS

This patent application claims priority to Chinese Patent Application No. CN 202311265343.6, filed on September 27, 2023, which is incorporated herein by reference in its entirety.

### FIELD OF THE INVENTION

The present invention relates to the field of antibody medicaments, and more specifically, the present invention relates to the use of an anti-tetanus toxin antibody in the prevention or treatment of tetanus.

### BACKGROUND OF THE INVENTION

Tetanus is a specific infection characterized by muscle spasms, which is caused by *Clostridium tetani* invading the human body, multiplying and producing toxins (tetanus toxin) in an anaerobic environment. Tetanus toxin primarily attacks motor neurons in the nervous system; therefore, the disease is clinically characterized by trismus, paroxysmal spasms, and tonic spasms. Globally, the mortality rate of tetanus is as high as 30%-50%, with the mortality rate for severe cases and newborns reaching as high as 80%. Although the incidence of tetanus is low in developed countries, it is still prevalent in low- and middle-income countries and underdeveloped regions, and is an important public health issue.

Proper wound care and the appropriate use of tetanus immunization agents after injury are crucial for preventing tetanus infection. Tetanus prevention methods include active immunization (*i.e.*, tetanus vaccine) and passive immunization (such as tetanus-specific immunoglobulin). The existing tetanus passive immunizing agents in China include human tetanus immunoglobulins (HTIGs), equine tetanus antitoxins (TATs), and equine tetanus immunoglobulin F(ab')₂. However, these preparations all have certain drawbacks. For example, equine tetanus antitoxins are mainly derived from equine serum, which pose a potential risk of hypersensitivity reactions to recipients, and a long immunization cycle. Although equine tetanus immunoglobulins have a lower allergy rate compared to TAT, they still cannot overcome the application barriers that are common to animal-derived therapeutic agents. On the other hand, human tetanus immunoglobulins are blood products, which pose a potential risk of infectious diseases such as AIDS and hepatitis B. Moreover, due to the complex industrial production and limited output, they cannot fully meet market demand. Furthermore, in clinical application, it has been found that the immune effect of these immune preparations has a short duration of action. Generally, equine tetanus antitoxins last only 9 days, equine tetanus immunoglobulins last 10 days, and human tetanus immunoglobulins (with a half-life of 25 days) can only maintain the effect for 2-3 weeks.

Therefore, there remains a need in the field for novel passive tetanus immunization agents for the prevention or treatment of tetanus.

### SUMMARY OF THE INVENTION

Tetanus toxin is highly toxic, acts rapidly, and has a high mortality rate. When a patient is diagnosed with tetanus infection, a large number of bacteria and toxins are usually present in his body. Given the various shortcomings of currently used tetanus passive immunizing agents, the effective approach is to promptly inject anti-tetanus toxin antibodies that are better in terms of efficacy (more quickly to achieve protective antibody levels, longer protection duration, and higher antibody titers), safety, quality controllability, and suitability for large-scale production.

The applicant of this application previously developed a recombinant native human monoclonal antibody against tetanus toxin, which is referred to as "TRN0011" in the present invention . The amino acid sequence of TRN0011 is as follows.

| | Amino acid sequence | SEQ ID NO |
|---|---|---|
| Heavy chain (HC) | QVQLQESGAGLVKPSETLSLTCGVAGGPFT GSYLSWIRQPPGKGLEWIGEVSQSGSTSYN PSLKSRVTISVDTSKSQFSLKLSSVTAADTAV YYCARLTKHYINSAYWGQGTLVTVSSASTK GPSVFPLAPSSKSTSGGTAALGCLVKDYFPEP VTVSWNSGALTSGVHTFPAVLQSSGLYSLSSV VTVPSSSLGTQTYICNVNHKPSNTKVDKRVEP KSCDKTHTCPPCPAPELLGGPSVFLFPPKPKDT LMISRTPEVTCVVVDVSHEDPEVKFNWYVDG VEVHNAKTKPREEQYNSTYRVVSVLTVLHQD WLNGKEYKCKVSNKALPAPIEKTISKAKGQP REPQVYTLPPSREEMTKNQVSLTCLVKGFYPS DIAVEWESNGQPENNYKTTPPVLDSDGSFFLY SKLTVDKSRWQQGNVFSCSVMHEALHNHYT QKSLSLSPGK | Heavy chain CDR1 (HCDR1): GGPFTGSY; SEQ ID NO. 1; |
| | | Heavy chain CDR2 (HCDR2): VSQSGST ; SEQ ID NO. 2; |
| | | Heavy chain CDR3 (HCDR3): ARLTKHYINSAY; SEQ ID NO. 3; |
| | | Heavy chain variable region (VH): |
| | | QVQLQESGAGLVKPSETLSLTCGVAGGPF TGSYLSWIRQPPGKGLEWIGEVSQSGSTS YNPSLKSRVTISVDTSKSQFSLKLSSVTAA DTAVYYCARLTKHYINSAYWGQGTLVTVS S; SEQ ID NO. 4; Heavy chain (HC): SEQ ID NO. 5 |
| Light chain (LC) | DIQMTQSPSILSASVGDRVTITCRASQIIGSW LAWYQQKPGKAPTLVIYKASRLDSGVPSRF SGTESGTEFTLTISSLQPDDFATYYCQQYNS YPYTFGQGTKLEIKRTVAAPSVFIFPPSDEQL KSGTASVVCLLNNFYPREAKVQWKVDNALQ SGNSQESVTEQDSKDSTYSLSSTLTLSKADYE KHKVYACEVTHQGLSSPVTKSFNRGEC | Light chain CDR1 (LCDR1): QIIGSW; SEQ ID NO. 6; |
| | | Light chain CDR2 (LCDR2): KAS; SEQ ID NO. 7; |
| | | Light chain CDR3 (LCDR3): QQYNSYPYT; SEQ ID NO. 8; |
| | | Light chain variable region (VL): |
| | | DIQMTQSPSILSASVGDRVTITCRASQIIGSW LAWYQQKPGKAPTLVIYKASRLDSGVPSRFS GTESGTEFTLTISSLQPDDFATYYCQQYNSYP YTFGQGTKLEIK ; SEQ ID NO. 9; Light chain (LC): SEQ ID NO. 10 |

"TRN0011" is an immunoglobulin G1 (IgG1) monoclonal antibody that can bind to a specific surface of tetanus toxin protein with high affinity, thereby neutralizing the toxin and preventing the enzymatic cleavage activity of tetanus toxin, thus protecting the body. By conducting *in vitro* and *in vivo* pharmacodynamic studies of TRN0011 in animals, and a randomized, double-blind, placebo-controlled, dose-escalation study on its safety, tolerability, pharmacokinetics and pharmacodynamics in healthy subjects, the applicant of the present application, with this antibody as the active agent, provides its use for the prevention or treatment of tetanus, as well as corresponding preventive or therapeutic regimens.

Specifically, the present invention provides the following technical solutions.

In one aspect, the present invention provides use of an anti-tetanus toxin antibody in the preparation of a medicament for the prevention or treatment of tetanus; the anti-tetanus toxin antibody comprises: heavy chain CDR1, heavy chain CDR2 and heavy chain CDR3 respectively comprising the amino acid sequences set forth in SEQ ID NO. 1, SEQ ID NO. 2 and SEQ ID NO. 3; and light chain CDR1, light chain CDR2 and light chain CDR3 respectively comprising the amino acid sequences set forth in SEQ ID NO. 6, SEQ ID NO. 7 and SEQ ID NO. 8.

Preferably, the anti-tetanus toxin antibody comprises: a heavy chain variable region comprising the amino acid sequence set forth in SEQ ID NO. 4; and a light chain variable region comprising the amino acid sequence set forth in SEQ ID NO. 9.

More preferably, the anti-tetanus toxin antibody comprises all or part of a heavy chain constant region and/or a light chain constant region.

According to a specific embodiment of the present invention, the anti-tetanus toxin antibody is a monoclonal antibody comprising two heavy chains and two light chains. Preferably, the anti-tetanus toxin antibody comprises: a heavy chain comprising the amino acid sequence set forth in SEQ ID NO. 5; and a light chain comprising the amino acid sequence set forth in SEQ ID NO. 10.

In the context of the present invention, the term "tetanus" refers to a specific infection characterized by muscle spasms, which is caused by *Clostridium tetani* invading the human body, multiplying and producing toxins (tetanus toxin) in an anaerobic environment. According to the 2020 edition of Clinical Practice Guidelines for Non-neonatal Tetanus issued by the National Health Commission of the People's Republic of China, the diagnosis of non-neonatal tetanus is mainly based on typical clinical manifestations, and requires at least one of the following manifestations: (1) trismus or risus sardonicus; and (2) painful muscle spasms. For patients with diagnostic uncertainty, the tongue depressor test may be performed by gently touching the posterior pharynx of the patient with a tongue depressor, with a positive result indicated by reflex spasm of the masseter muscle rather than a normal gag reflex. Tetanus can cause temporary functional changes in the central nervous system, manifested as tonic contraction and paroxysmal spasms of skeletal muscles throughout the body. Specifically, the symptoms of tetanus include spasms of the facial expression muscles, neck, back, abdomen, limb, and diaphragmatic muscles.

According to the present invention, the anti-tetanus toxin antibody is used to prepare a medicament that can prevent or treat tetanus, and the medicament can be administered (*e.g.*, by injection) to a subject to prevent or treat tetanus. The medicament is used in a subject who has been infected with, or has not yet been infected with, *Clostridium tetani*. In theory, any wound or break may expose a subject to *Clostridium tetani* infection, and prophylaxis is therefore required. Common causes include: (1) history of skin or mucosal trauma or damage (such as animal bites, drug injection, childbirth or abortion); (2) history of bacterial infections of skin, mucosa or soft tissues (such as chronic otitis media, chronic sinusitis, periodontal infection, *etc.*); (3) history of gastrointestinal damage (such as gastrointestinal surgery).

The subjects are mammals that have been infected or have not been infected with *Clostridium tetani*. In the context of the present invention, the term "mammal" means any animal classified as a mammal, including but not limited to humans, livestock and agricultural animals, as well as zoo, sport or pet animals, such as sheep, dogs, horses, cats, cattle, rats, pigs, apes such as macaques, *etc*. Preferably, the mammal is a primate, and more preferably a human. In the case that the subject is human, an adult aged 18 years or older is preferred.

Tetanus is prevented or treated by administering the medicament to the subject in a single dose. Preferably, the medicament is an intramuscular injection preparation for administration of the medicament.

The antibody dose achieved by administering the medicament ranges from 0.6 mg to 15 mg, for example 0.6 mg, 1.8 mg, 2.1 mg, 5 mg, 6 mg, 10 mg or 15 mg; preferably, the antibody dose achieved ranges from 5 mg to 15 mg.
More preferably, the antibody dose achieved is 10 mg.

Alternatively, based on the average weight of the subject being 60 kg, the antibody dose achieved by administering the medicament ranges from 10 µg/kg to 250 µg/kg, for example, 10 µg/kg, 30 µg/kg, 35 µg/kg, 83 µg/kg, 100 µg/kg, 167 µg/kg, or 250 µg/kg; preferably, the antibody dose achieved ranges from 83 µg/kg to 250 µg/kg. More preferably, the antibody dose achieved is 167 µg/kg.

The administration of the above-mentioned medicaments can achieve an anti-tetanus toxin neutralizing antibody titer ≥ 0.01 IU/mL in the subject.

Preferably, the anti-tetanus toxin neutralizing antibody titer is detectable in the serum of the subject; more preferably, the medicament is used to achieve an anti-tetanus toxin neutralizing antibody titer ≥ 0.01 IU/mL in the subject within 48 h, preferably 24 h, more preferably 12 h, further preferably 6 h, or even less after administration.

The administration of the above-mentioned medicament can achieve an anti-tetanus toxin neutralizing antibody titer lasting for more than 90 days, preferably lasting for more than 105 days, in the subject.

In the aspect of the use of the antibody in the preparation of a medicament for the prevention or treatment of tetanus as described in the invention, the above-mentioned features ( *e.g.*, dosage, route of administration, frequency of administration, dosing cycle, *etc.*) can be arbitrarily selected and combined. For example, according to a specific embodiment of the present invention, the present invention provides the use of the anti-tetanus toxin antibody in the preparation of a medicament for the prevention or treatment of tetanus, the medicament being administered to the subject by a single intramuscular injection to achieve an antibody dose of 10 mg.

In another aspect, the present invention provides an anti-tetanus toxin antibody, which is used as a preventive or therapeutic agent for tetanus, and is therefore used in a method comprising administering the antibody to a subject in need of preventing or treating tetanus. The anti-tetanus toxin antibody comprises: heavy chain CDR1, heavy chain CDR2 and heavy chain CDR3 respectively comprising the amino acid sequences set forth in SEQ ID NO. 1, SEQ ID NO. 2 and SEQ ID NO. 3; and light chain CDR1, light chain CDR2 and light chain CDR3 respectively comprising the amino acid sequences set forth in SEQ ID NO. 6, SEQ ID NO. 7 and SEQ ID NO. 8.

Preferably, the anti-tetanus toxin antibody provided by the present invention comprises: a heavy chain variable region comprising the amino acid sequence set forth in SEQ ID NO. 4 ; and a light chain variable region comprising the amino acid sequence set forth in SEQ ID NO. 9 .

More preferably, the anti-tetanus toxin antibody comprises all or part of a heavy chain constant region and/or a light chain constant region.

According to a specific embodiment of the present invention, the anti-tetanus toxin antibody is a monoclonal antibody comprising two heavy chains and two light chains. Preferably, the anti-tetanus toxin antibody comprises: a heavy chain comprising the amino acid sequence set forth in SEQ ID NO. 5; and a light chain comprising the amino acid sequence set forth in SEQ ID NO. 10.

The term "tetanus" is as defined above. In particular, the tetanus symptoms treated by the anti-tetanus toxin antibody provided by the present invention are spasms of facial expression muscles, neck, back, abdomen, limb and diaphragmatic muscles.

The anti-tetanus toxin antibody provided by the present invention is used in a method for preventing or treating tetanus, the method including administering (*e.g.*, injecting) the antibody to a subject to prevent or treat tetanus. The subjects are mammals that have infected with or have not been infected with *Clostridium tetani*.

The term "mammal" is as defined above. Preferably, the mammal is a primate, and more preferably a human. Where the subject is human, an adult is preferred, such as an adult aged 18 years or older.

Preferably, the method includes administering the anti-tetanus toxin antibody to the subject in a single dose to prevent or treat tetanus; preferably, the anti-tetanus toxin antibody is administered via intramuscular injection.

The antibody dose administered in the method ranges from 0.6 mg to 15 mg, for example 0.6 mg, 1.8 mg, 2.1 mg, 5 mg, 6 mg, 10 mg or 15 mg; preferably, the antibody dose ranges from 5 mg to 15 mg; more preferably, the antibody dose is 10 mg.

Alternatively, based on the average weight of the subject being 60 kg, the antibody dose administered in the method can be 10-250 µg/kg, for example 10 µg/kg, 30 µg/kg, 35 µg/kg, 83 µg/kg, 100 µg/kg, 167 µg/kg or 250 µg/kg; preferably, the antibody dose ranges from 83 µg/kg to 250 µg/kg; more preferably, the antibody dose is 167 µg/kg.

The above method can achieve an anti-tetanus toxin neutralizing antibody titer ≥ 0.01 IU/mL in the subject.

Preferably, the anti-tetanus toxin neutralizing antibody titer is detectable in the serum of the subject; more preferably, the method includes achieving an anti-tetanus toxin neutralizing antibody titer ≥ 0.01 IU/mL in the subject within 48 h, preferably 24 h, more preferably 12 h, further preferably 6 h, or even less after administration.

The above method can achieve an anti-tetanus toxin neutralizing antibody titer lasting for more than 90 days, preferably lasting for more than 105 days, in the subject.

In the aspect of the use of the antibody as a preventive or therapeutic agent for tetanus as described in the invention, the defined administration characteristics (*e.g.*, dosage, route of administration, frequency of administration, dosing cycle, *etc.*) can be arbitrarily selected and combined. For example, according to a specific embodiment of the present invention, the present invention provides the antibody for use as a preventive or therapeutic agent for tetanus, or for use in a method for preventing or treating tetanus, comprising a single intramuscular injection of 10 mg of the anti-tetanus toxin antibody into the subject .

In another aspect, the present invention provides a method for preventing or treating tetanus, the method including administering the anti-tetanus toxin antibody to a subject in need thereof. The anti-tetanus toxin antibody is as defined above.

The term "tetanus" is as defined above. Specifically, the symptoms of tetanus include spasms of the facial expression muscles, neck, back, abdomen, limb, and diaphragmatic muscles.

The method for preventing or treating tetanus provided by the present invention may include administering (*e.g.*, injecting) the antibody to a subject to prevent or treat tetanus, wherein, the subjects are mammals that have infected with or have not been infected with *Clostridium tetani*. For example, the subject belongs to a population that has not completed a full course of tetanus immunization or has an unknown immunization history.

The term "mammal" is as defined above. Preferably, the mammal is a primate, and more preferably a human. Where the subject is human, an adult is preferred, such as an adult over 18 years of age.

Preferably, the method includes administering the anti-tetanus toxin antibody to the subject in a single dose to prevent or treat tetanus; preferably, the anti-tetanus toxin antibody is administered via intramuscular injection.

The antibody dose administered in the method is 0.6-15 mg, for example 0.6 mg, 1.8 mg, 2.1 mg, 5 mg, 6 mg, 10 mg or 15 mg; preferably, the antibody dose is 5-15 mg; more preferably, the antibody dose is 10 mg.

Alternatively, based on the average weight of the subject being 60 kg, the antibody dose administered in the method can be 10-250 µg/kg, for example 10 µg/kg, 30 µg/kg, 35 µg/kg, 83 µg/kg, 100 µg/kg, 167 µg/kg or 250 µg/kg; preferably, the antibody dose ranges from 83 µg/kg to 250 µg/kg; more preferably, the antibody dose is 167 µg/kg.

The above method can achieve an anti-tetanus toxin neutralizing antibody titer ≥ 0.01 IU/mL in the subject.

Preferably, the anti-tetanus toxin neutralizing antibody titer is detectable in the serum of the subject; more preferably, the method includes achieving an anti-tetanus toxin neutralizing antibody titer ≥ 0.01 IU/mL in the subject within 48 h, preferably 24 h, more preferably 12 h, further preferably 6 h, or even less after administration.

The above method can achieve an anti-tetanus toxin neutralizing antibody titer lasting for more than 90 days, preferably lasting for more than 105 days, in the subject.

In the aspect of the use of the antibody in the preventive or therapeutic method for tetanus as described in the invention, the defined administration characteristics (*e.g.*, dosage, route of administration, frequency of administration, dosing cycle, *etc.*) can be arbitrarily selected and combined. For example, according to a specific embodiment of the present invention, the present invention provides a method for preventing or treating tetanus, the method including administering a single intramuscular injection of 10 mg of the anti-tetanus toxin antibody to the subject .

By conducting *in vitro* and *in vivo* pharmacodynamic studies in animals, as well as randomized, double-blind, placebo-controlled, dose-escalation studies in healthy subjects on safety, tolerability, pharmacokinetics, and pharmacodynamics with the previously provided immunoglobulin G1 (IgG1) monoclonal antibody, the applicant of the invention determined an optimal antibody application regimen suitable for the prevention or treatment of tetanus in humans.

Studies have shown that the tetanus monoclonal antibody TRN0011, administered to subjects using a specific dosing regimen, can achieve a faster onset of action and a longer duration of protection than human tetanus immunoglobulin (HTIG), and therefore can effectively prevent or treat tetanus.

Specifically, the literature, relevant guidelines, and previous studies indicate that, whether passive immunization or active immunization is used, immunoprotective efficacy can be achieved as long as the serum level of anti-tetanus toxin neutralizing antibodies in the subject reaches 0.01 IU/mL. The marketed human tetanus immunoglobulin achieves a serum antibody level exceeding 0.01 IU/mL at 3 days after injection (see Rongsheng Yipu HTIG Package Insert, Clinical Studies). In contrast, based on a comprehensive analysis of the results from various studies, it can be determined that, following intramuscular administration of TRN0011, the serum titer of anti-tetanus toxin neutralizing antibodies increases to the minimum protective level of 0.01 IU/mL as early as 6 hours after administration, or even within a shorter time period, and the magnitude of the increase in neutralizing antibody titer rises with increasing administered dose.

Moreover, with respect to the duration of protective immunity, intramuscular administration of TRN0011 is capable of providing anti-tetanus protection to the majority (>90%) of treated subjects as early as 12 hours after administration, and such protection is maintained until 105 days post-administration. Accordingly, compared with human tetanus immunoglobulin, TRN0011 provides anti-tetanus protection at an earlier time point and for a longer duration, with higher antibody titer levels. Furthermore, considering that the anti-tetanus toxin neutralizing antibody titer is still detectable at Day 106 after administration, which is far above the minimum protection threshold, it can be concluded that administration of TRN0011 can provide protective effect for a much longer duration.

Furthermore, based on a comprehensive analysis of the results from various studies, it can be determined that TRN0011 exhibits low immunogenicity and does not affect the pharmacokinetic profile or safety outcomes in individuals, and that TRN0011 has good safety and tolerability.

Accordingly, without being bound by any theory, it is expected that the anti-tetanus toxin antibody described herein is capable of achieving improved prophylactic or therapeutic effects against tetanus.

### DESCRIPTION OF THE DRAWINGS

The embodiments of the present invention will now be described in detail with reference to the accompanying drawings, wherein:
Figure 1 shows the proportion of subjects in Example 1 whose anti-tetanus toxin neutralizing antibody titer increased from baseline (ΔTiter) to ≥ 0.01 IU/mL.
Figure 2 shows the Kaplan-Meier curve of the duration of the increase in anti-tetanus toxin neutralizing antibody titer from baseline (ΔTiter) to ≥ 0.01 IU/mL in Example 1.
Figure 3 shows the time-dependent trend of the increase in anti-tetanus toxin neutralizing antibody titer from baseline (ΔTiter) in Example 1.

### DETAILED EMBODIMENT OF THE INVENTION

The present invention will be described below with reference to specific embodiments. Those skilled in the art will understand that these embodiments are for illustrative purposes only and do not limit the scope of the invention in any way.

Unless otherwise specified, the experimental methods described in the following examples are conventional methods. Unless otherwise specified, all raw materials, reagents, and other materials used in the following examples are commercially available products.

The antibody used in the following examples is a recombinant native human monoclonal antibody against tetanus toxin, TRN0011 (hereinafter referred to as "antibody" or "the previously referred antibody" in the context of the present invention), and the amino acid sequence and other characteristics of the antibody are as defined above.

The test medicament in the following examples is an injection containing TRN0011; or a placebo containing the same excipients as the injection but without the antibody.

Regardless of the specific dosage, the groups that receive TRN0011 are collectively referred to as the " TRN0011 group".

The titer of anti-tetanus toxin neutralizing antibodies was detected in serum using enzyme-linked immunosorbent assay (ELISA) .

The HTIG control used in the following examples is an immunoglobulin from Hualan Biological Engineering Co., Ltd.

In the following examples, "subject" and "volunteer" are interchangeable.

### EXAMPLE 1

A multicenter, randomized, double-blind, parallel-controlled, dose-finding study was conducted in Chinese adult volunteers to compare the anti-tetanus toxin neutralizing antibody titers and safety after a single intramuscular injection of TRN0011 antibody, human tetanus immunoglobulin (HTIG), or placebo.

Trial objective: The primary objective was to compare the anti-tetanus toxin neutralizing antibody titers after a single intramuscular injection (IM) of TRN0011 and human tetanus immunoglobulin (HTIG) in Chinese adult volunteers. The secondary objective was to compare the safety and tolerability after a single intramuscular injection of TRN0011, HTIG and placebo in Chinese adult volunteers; to assess the pharmacokinetic (PK) characteristics of TRN0011 in Chinese adult volunteers; and to assess the immunogenicity of TRN0011 injection in Chinese adult volunteers.

### (i) Study Design

The study included a screening period (Day -28 to Day -2), a hospital stay period (Day -1 to Day 4), and a follow-up period (Day 5 to Day 106).

Eligible volunteers were admitted to the study center on Day -1. After completing the protocol-required assessments and randomization, the volunteers received administration of the investigational medicinal product on Day 1. Preparation and administration of the investigational medicinal product were performed by unblinded study personnel, and the injection time for all investigational medicinal products was controlled at 30 (±3) seconds.

After dosing, the volunteers remained at the study center for safety assessments, including physical examinations, vital signs, 12-lead electrocardiograms (ECGs), and laboratory tests (hematology, blood biochemistry, and urinalysis). Meanwhile, serum samples were collected by researchers as scheduled for analysis of anti-tetanus toxin neutralizing antibody titers, pharmacokinetics (PK), and immunogenicity.

### (ii) Study Population

Considering the severe clinical consequences associated with failure of tetanus prophylaxis, dose finding was not considered appropriate in a trauma population. Meanwhile, given that most individuals in the target population for tetanus, apart from trauma, are generally in a healthy condition, this study enrolled healthy volunteers or volunteers with chronic diseases in a stable condition.

Study participants were selected according to the following criteria.

### Inclusion Criteria:

1) Chinese adults (aged ≥ 18 years), male or female;
2) Healthy volunteers or volunteers with stable chronic diseases who had normal findings or abnormalities without clinical significance in physical examination, vital signs, and clinical laboratory tests during the screening period, and who were judged by the investigator to be unlikely to be exposed to significant risk by participating in the clinical study.

### (III) Study Scheme

A total of 240 Chinese adult volunteers were enrolled.

The volunteers were randomized in a 2:2:1:2:1 ratio into the following groups: TRN0011 5 mg group (0.25 mL), TRN0011 10 mg group (0.5 mL), TRN0011 15 mg group (0.75 mL), HTIG 250 IU group (2.5 mL), and placebo group (0.5 mL). All subjects received a single intramuscular injection into the gluteal muscle.

Among the above groups, the doses of TRN0011 were determined based on results from previous studies. HTIG is the preferred passive immunizing agent recommended by current domestic and international guidelines for tetanus prophylaxis and has well-established protective efficacy against tetanus, with long-term and widespread clinical use. A single intramuscular injection of HTIG at a dose of 250 IU is the standard clinical dose for tetanus prophylaxis. Accordingly, HTIG was selected as the positive control at a dose of 250 IU. In addition, a placebo was selected as a control to comprehensively evaluate the safety of TRN0011.

### (iv) Evaluation of study indicators

### Primary endpoint:

- Proportion of volunteers with an increase of anti-tetanus toxin neutralizing antibody titer from baseline (ΔTiter) ≥ 0.01 IU/mL at 24 hours post-dose (in this study, serum anti-tetanus toxin neutralizing antibody titer was used as a pharmacodynamic parameter).

The incubation period of tetanus is generally 3-21 days after infection, with a median of 7 days; however, onset may occur as late as 178 days or as early as 1-2 days after infection, the latter mainly being associated with cephalic tetanus. Therefore, a product intended for tetanus prophylaxis should provide protective efficacy within 24 hours after injury.

According to World Health Organization (WHO) Guidelines and previous studies, the level of anti-tetanus toxin neutralizing antibody titers in the body following the administration of active or passive immunizing agents correlates with protective efficacy. In the list of surrogate endpoints recommended by the U.S. Food and Drug Administration (FDA) for drug approval and licensure, the immunological surrogate endpoint for tetanus vaccines is the level of anti-tetanus toxin antibodies.

Existing evidence indicates that an anti-tetanus toxin neutralizing antibody level exceeding 0.01 IU/mL, as measured by *in vivo* neutralization assays or modified enzyme-linked immunosorbent assays (ELISA), is generally considered protective. Taking into account the disease course of tetanus infection and the possibility that the study population may have pre-existing antibodies due to prior active immunization against tetanus with tetanus vaccines, the primary endpoint of this study was defined as the proportion of volunteers whose anti-tetanus toxin neutralizing antibody titer increased from baseline by ≥ 0.01 IU/mL at 24 hours post-dose.

### Secondary endpoint:

- Safety endpoints, including the type and incidence of adverse events and serious adverse events, as well as results of physical examinations, vital signs, 12-lead ECGs, and laboratory tests (hematology, blood biochemistry, and urinalysis);
- Change from baseline in anti-tetanus toxin neutralizing antibody titers at 24 hours, 48 hours (Day 3), and Days 7, 21, 30, and 90 post-dose;
- Proportion of volunteers with anti-tetanus toxin neutralizing antibody ΔTiter ≥ 0.01 IU/mL at 48 hours (Day 3), and Days 7, 21, 30, and 90 post-dose.
- Proportion of volunteers with anti-tetanus toxin neutralizing antibodyΔTiter ≥ 0.01 IU/mL at both 24 hours and Day 30 post-dose.
- Duration of anti-tetanus toxin neutralizing antibody ΔTiter ≥ 0.01 IU/mL post-dose;
- Anti-tetanus toxin neutralizing antibody titers at 24 hours, 48 hours (Day 3), and Days 7, 21, 30, and 90 post-dose;
- Proportion of volunteers with anti-tetanus toxin neutralizing antibody titer ≥ 0.01 IU/mL at 24 hours, 48 hours (Day 3), and Days 7, 21, 30, and 90 post-dose;
- Pharmacokinetic parameters of TRN0011. Primary PK parameters included maximum serum concentration (Cₘₐₓ), time to maximum concentration (Tₘₐₓ), elimination half-life (t_{1/2}), area under the plasma concentration-time curve from time 0 to t (AUC₀₋ₜ), and area under the plasma concentration-time curve from time 0 to infinity (AUC_{0-∞}). If data permitted, apparent clearance (CL/F) and apparent volume of distribution (V_{d}/F) were also included.
- Positive rate of anti-drug antibodies (ADA) in volunteers in TRN0011 group.

### (v) Statistical methods

### Analysis sets

- Full Analysis Set (FAS): All randomized volunteers who received at least one dose of the investigational medicinal product would serve as FAS. The FAS was used for analyses of volunteer disposition, demographics, and baseline characteristics.
- Anti-tetanus Toxin Neutralizing Antibody Titer Analysis Set: All randomized volunteers who received at least one dose of the investigational medicinal product and had at least one valid post-dose serum anti-tetanus toxin neutralizing antibody titer measurement were included in this analysis set. If events occurred that could affect neutralizing antibody titers or their assessment (*e.g.*, significant protocol deviations related to eligibility criteria, use of prohibited medications during the study), all or part of the antibody data from the affected volunteers were excluded from this analysis set.
- Safety Analysis Set (SS): All randomized volunteers who received at least one dose of the investigational medicinal product and had at least one post-dose safety assessment were included in the SS. SS is the population included in the safety evaluation of this study.

- PK Concentration Analysis Set (PKCS): All randomized volunteers who received at least one dose of the investigational medicinal product and had at least one analyzable PK concentration point during the trial period were included in the PKCS of this study. If events occurred that could affect PK concentration results or their assessment (*e.g.*, significant protocol deviations related to eligibility criteria, use of prohibited medications during the study ), all or part of the PK data from the affected volunteers were excluded from PKCS. The PK Concentration Analysis Set will be used for pharmacokinetic concentration analysis.
- PK Parameter Analysis Set (PKPS): All randomized volunteers who received at least one dose of the investigational medicinal product and had at least one analyzable PK parameter during the trial period. If events occurred that could affect PK parameter results or their assessment (*e.g.*, significant protocol deviations related to eligibility criteria, use of prohibited medications during the study), all or part of the PK data from the affected volunteers were excluded from PKPS. The PK Parameter Analysis Set will be used for pharmacokinetic parameter analysis.
- Immunogenicity Analysis Set: All randomized volunteers who received at least one dose of TRN0011 and had at least one post-dose immunogenicity data were included in this set.

### Basic principles

The PK parameters were calculated using Phoenix WinNonlin version 8.3.1, and all other statistical analyses were performed using Statistical Analysis System (SAS) version 9.4.

Analysis of anti-tetanus toxin neutralizing antibody titers, pharmacokinetics, immunogenicity, and safety was based on the actual administration received by volunteers. Other analyses were based on randomized administration groups of volunteers unless otherwise specified.

### Analysis of anti-tetanus toxin neutralizing antibody titers

Analysis of anti-tetanus toxin neutralizing antibody titers was based on Anti-tetanus Toxin Neutralizing Antibody Titer Analysis Set.

According to the dosing group, the number and percentage of volunteers with a ΔTiter ≥0.01 IU/mL at 24 hours post-dose were calculated, together with the corresponding 95% CI and 90% CI (based on the Clopper-Pearson method). In addition, the difference between groups in the proportion of volunteers with ΔTiter ≥0.01 IU/mL was provided, along with the 95% CI and 90% CI of the difference (based on the stratified Miettinen-Nurminen method. Stratification was performed based on the results of the rapid tetanus antibody IgG test at the time of random stratification).

In addition, based on the baseline tetanus toxin-neutralizing antibody titers of the volunteers (as measured in serum samples by a chemiluminescence assay), all endpoints related to tetanus toxin-neutralizing antibody titers were analyzed in subgroups using 0.01 IU/mL (<0.01 IU/mL and ≥0.01 IU/mL) as the cutoff value.

A sensitivity analysis was performed using the Newcombe-Wilson score to calculate the 95% CI and 90% CI for the difference in the proportion of volunteers with anti-tetanus toxin-neutralizing antibody ΔTiter ≥0.01 IU/mL among groups.

### Pharmacokinetic analysis

### • PK concentration analysis

PK concentrations of the investigational medicinal product were summarized by dosing group and scheduled sampling time points, including the number of volunteers, the number of volunteers with concentrations below the lower limit of quantification (BLQ), arithmetic mean, SD, median, minimum, maximum, CV (based on the arithmetic mean), and geometric mean.

Mean drug concentration-time profiles were plotted according to the scheduled sampling times on both linear and semi-logarithmic scales.

### • PK parameter analysis

PK parameters (Cₘₐₓ, AUC₀₋ₜ, AUC_{0-∞}, Tₘₐₓ, t_{1/2}, V_{d}/F, CL/F, *etc.*) were calculated using noncompartmental analysis (NCA).

PK parameters were summarized according to dosing group, including the number of volunteers, arithmetic mean, SD, median, minimum, and maximum. For PK parameters other than Tₘₐₓ, the CV (based on the arithmetic mean), geometric mean, geometric standard deviation, and geometric coefficient of variation were also calculated.

Individual PK parameters for all randomized volunteers were listed according to dosing group.

### Immunogenicity analysis

Based on the actual dosing group, the number and percentage of volunteers who were ADA-positive at least once during the trial period, ADA-positive at baseline, ADA-positive at least once post-baseline, and ADA-positive at each post-baseline visit were summarized.

Immunogenicity assay results at each time point were tabulated according to dosing group.

### Safety Analysis

Safety analyses were summarized by actual dosing group and dosage. The safety analysis included descriptive statistics and individual listings for all safety data, including adverse events, physical examinations, vital signs, 12-lead ECGs, clinical laboratory assessments, and injection-site evaluations.

Adverse events were coded using the Medical Dictionary for Regulatory Activities (MedDRA), Version 25.1. Treatment-emergent adverse events (TEAEs) were defined as adverse events (AE) that occurred after administration of the investigational medicinal product or as a worsening of pre-existing medical conditions.

### Analyses conducted

### • Primary Endpoint Analysis

The primary endpoint analysis was performed after all volunteers had obtained primary endpoint data and safety data from the Day 30 visit. This analysis also included all safety data and other secondary endpoint data available at the time of analysis.

### • Updated Analysis of Safety Endpoints and Other Secondary Endpoints

An updated analysis of safety endpoints and other secondary endpoints was conducted at the end of the study.

### (vi) Study Results

### 1. Volunteer Disposition and Characteristics

A total of 240 adult volunteers were enrolled in this study, including 151 in the TRN0011 group (61 in the 5 mg group, 61 in the 10 mg group, and 29 in the 15 mg group), 59 in the HTIG group, and 30 in the placebo group. Overall, 7 volunteers discontinued the study prematurely, including 3 in the TRN0011 5 mg group, 2 in the 10 mg group, 1 in the 15 mg group, and 1 in the HTIG group. Among these, 6 volunteers (5 in the TRN0011 group and 1 in the HTIG group) withdrew from the study prematurely due to loss to follow-up, and 1 volunteer (TRN0011 5 mg group) withdrew from the study prematurely due to inability to attend follow-up visits.
See Table 1.

**Table 1.Volunteer disposition (all volunteers)**

| | **5mg (N=61) n(%)** | **10mg (N=61) n(%)** | **15mg (N=29) n(%)** | **TRN0011 (N=151) n(%)** | **HTIG (N=59) n(%)** | **Placebo (N=30) n(%)** | **Total (N=240) n(%)** |
|---|---|---|---|---|---|---|---|
| Randomiza tion | 61(100) | 61(100) | 29(100) | 151(100) | 59(100) | 30(100) | 240(100) |
| Completed study | 58(95.1) | 59(96.7) | 28(96.6) | 145(96.0) | 58(98.3) | 30(100) | 233(97.1) |
| Early withdrawal | 3(4.9) | 2(3.3) | 1(3.4) | 6(4.0) | 1(1.7) | 0 | 7(2.9) |
| Loss to follow-up | 2(3.3) | 2(3.3) | 1(3.4) | 5(3.3) | 1(1.7) | 0 | 6(2.5) |
| Other reasons | 1(1.6) | 0 | 0 | 1(0.7) | 0 | 0 | 1(0.4) |

Percentages were calculated based on all randomized volunteers in each actual dosing group.

A total of 240 volunteers were included in the FAS and SS sets. All volunteers in the TRN0011 and HTIG groups (a total of 210 cases) were included in the Anti-tetanus Toxin Neutralizing Antibody Titer Analysis Set. All volunteers in the TRN0011 groups (a total of 151 cases) were included in the PKCS, PKPS, and Immunogenicity Analysis Set.

### 2. Anti-Tetanus Toxin Neutralizing Antibody Titer Results

### 2.1 Primary endpoint

At 24 hours post-dose, the proportion of volunteers achieving anti-tetanus toxin neutralizing antibody ΔTiter ≥0.01 IU/mL was 91.8%, 100%, and 100% in the TRN0011 5 mg, 10 mg, and 15 mg groups, respectively, all higher than in the HTIG group (81.4%). No significant differences were observed between the TRN0011 dose groups. The differences between the TRN0011 dosing groups (5 mg, 10 mg, and 15 mg) and the HTIG group were 10.4% (95% CI: -1.2, 24.3), 18.6% (95% CI: 10.1, 31.0), and 18.6% (95% CI: 1.7, 30.0), respectively.

The lower limits of the 95% CI for the percentage differences between the TRN0011 group (10 mg, 15 mg) and the HTIG group were all >0, and the lower limits of the 90% CI for the percentage differences between all TRN0011 dosing groups and the HTIG group were all >0.

The results are shown in Table 2.

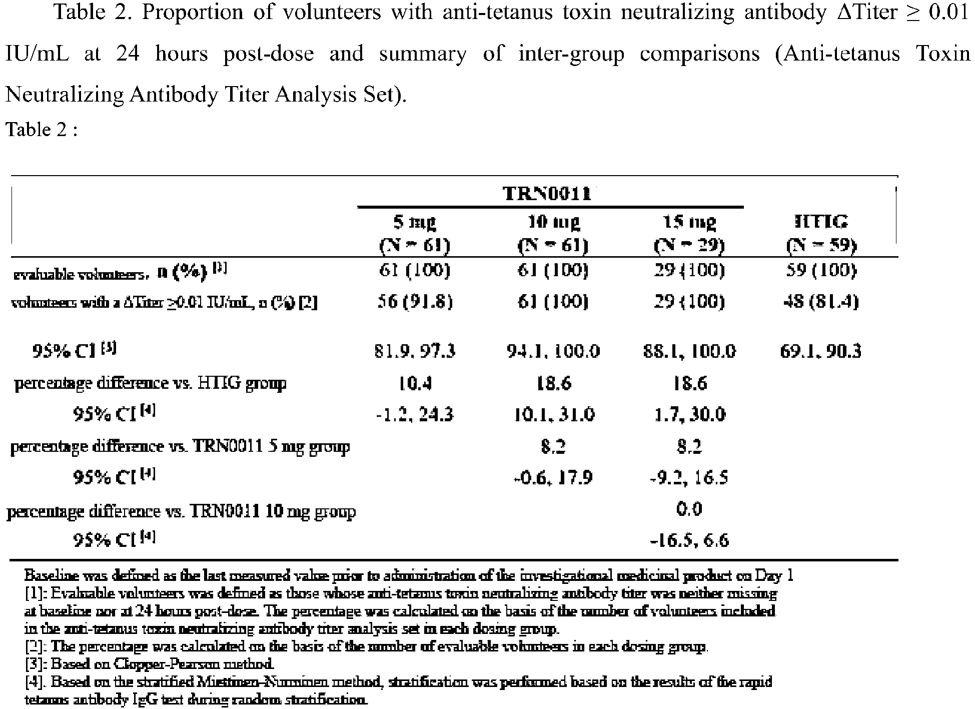

### 2.2 Secondary endpoints

For the secondary endpoints, the following results were obtained.

2.2.1 At 6 hours post-dose, the proportion of volunteers with ΔTiter ≥0.01 IU/mL in the TRN0011 5 mg, 10 mg, and 15 mg groups reached 55.7%, 80.3%, and 96.6%, respectively, and reached 100% at 72, 48, and 12 hours, respectively. In contrast, the proportion of volunteers with ΔTiter ≥0.01 IU/mL in HTIG group reached only 20.3% at 6 hours and did not reach 100% until Day 7.

2.2.2 At 12 hours post-dose, the proportion of volunteers achieving ΔTiter ≥0.01 IU/mL was 75.4%, 91.8% and 100% in the TRN0011 5 mg, 10 mg, and 15 mg groups, respectively, all higher than the proportion in the HTIG group (55.9%). The intergroup differences between each TRN0011 dosing group and the HTIG group were 19.5%, 35.9%, and 44.1%, respectively, with the lower limits of the 95% CIs all above 0.

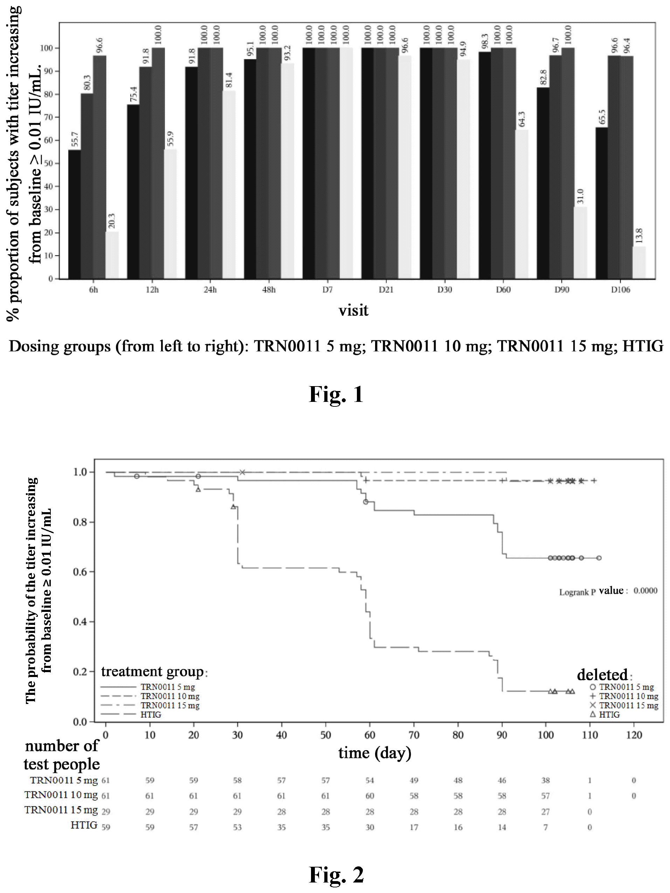

**2.2.3** From 48 hours to Day 30, the proportion of volunteers with ΔTiter ≥0.01 IU/mL remained high (>90.0%) for each TRN0011 dosing group and the HTIG group. Subsequently, the proportion of volunteers with ΔTiter ≥0.01 IU/mL in TRN0011 10mg and 15mg groups continued to maintain high levels, and on day 106, the proportions of the two groups remained at 96.6% and 96.4%, respectively. However, in the HTIG group, the proportion of volunteers with ΔTiter ≥0.01 IU/mL dropped significantly to 64.3% on Day 60, and further decreased to 13.8% on Day 106. The results are shown in Figure 1.

**2.2.4** When analyzing the proportion of volunteers with ΔTiter of ≥0.01 IU/mL at both 24 hours and Day 30, the proportions in the TRN0011 5 mg, 10 mg and 15 mg groups were 91.5%, 100% and 100%, respectively, all of which were higher than that in the HTIG group (78.0%).

**2.2.5** In the TRN0011 5 mg, 10 mg and 15 mg groups, peak values of ΔTiter were observed at Days 7-14, with the geometric mean titers (GMTs) of 0.1362, 0.2801, and 0.4786 IU/mL, respectively. The increase in ΔTiter showed an essentially linear relationship with the rise in administration dose. Similar to TRN0011, the peak value of ΔTiter in the HTIG group was observed on Day 7 post-dose, but the mean titer (0.0392 IU/mL) was significantly lower than those in all TRN0011 dosing groups.

**2.2.6** The GMT of ΔTiter in all TRN0011 dosing groups remained ≥0.01 IU/mL from 6 hours to Day 106 post-dose, whereas GMT of ΔTiter ≥0.01 IU/mL in the HTIG group stayed from 12 hours post-dose to Day 60.

**2.2.7** At each time point after dosing, the ΔTiter GMT of the TRN0011 groups was higher than that of the HTIG group.

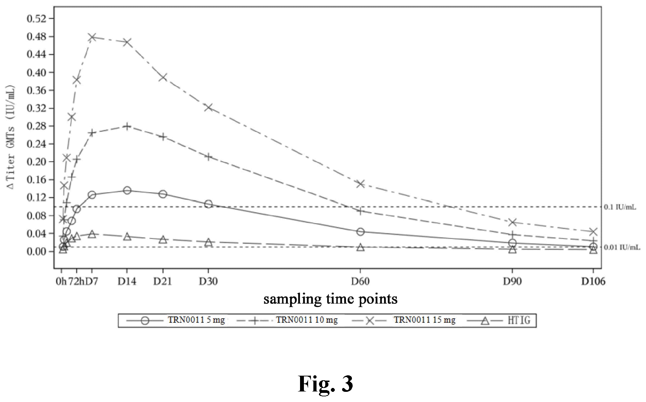

The Kaplan-Meier curves for the duration of anti-tetanus toxin neutralizing antibody titer increase from baseline (ΔTiter) ≥0.01 IU/mL in each treatment group (Anti-tetanus Toxin Neutralizing Antibody Titer Level Analysis Set) are presented in Figure 2; the time-trend curves of anti-tetanus toxin neutralizing antibody titer increase from baseline (ΔTiter) post-dose (Anti-tetanus Toxin Neutralizing Antibody Titer Level Analysis Set) are presented in Figure 3.

### 3. Pharmacokinetics

The results demonstrated that after a single intramuscular injection of TRN0011 within the dose range of 5-15 mg, the serum drug exposure (AUC₀₋ , AUC₀₋∞ and Cₘₐₓ) increased with the elevation of the administered dose, and the increase in drug exposure was approximately linear with respect to the increase in dosage.

The median time to peak serum drug concentration (Tₘₐₓ) of TRN0011 was 6.0-13.0 days. After reaching the peak, the serum drug concentration decreased over time, with a mean terminal elimination phase half-life (t_{½}) of 25.1-26.8 days.

No apparent differences were observed in the mean volume of distribution or clearance among the TRN0011 5 mg, 10 mg, and 15 mg groups. The mean V_{d}/F ranged from 7,320 to 8,570 mL, and the mean CL/F ranged from 8.17 to 10.2 mL/h.

### 4. Immunogenicity

The results showed that among the 151 volunteers who received TRN0011, only 2.65% (4/151) of the volunteers had TRN0011-related immunogenic reaction, *i.e.*, the ADA test result was positive. The highest titer of the ADA positive samples was 6.14, and none of them had neutralizing activity. Furthermore, it was found that immunogenicity did not have a obvious effect on individual PK and safety outcomes.

### 5. Safty

Key safety results are summarized as follows:
Among all volunteers who received TRN0011, 48.3% (73/151) experienced at least one TEAE, which was slightly lower than that in the HTIG group (54.2%, 32/59) and the placebo group (53.3%, 16/30). The incidence of TEAE was similar across all TRN0011 dosing groups, and no dose-related trend was observed.

Almost all cases of TEAE were mild or moderate in severity. The most common TEAEs were blood triglycerides increased and blood uric acid increased. The incidence of these two types of TEAEs was similar in the TRN0011 group and the placebo group, at 10.6% vs 16.7% and 8.6% vs 6.7%, respectively.

During the trial, no serious adverse events (SAEs) were reported in volunteers who received TRN0011 or placebo. One case of SAE (subdural hemorrhage) was reported in a volunteer who received HTIG. The severity of the SAE was severe and was determined by the investigators to be unrelated to the investigational medicinal product.

During the trial, no volunteers died or withdrew from the study prematurely due to TEAE.

### 6. Conclusion

In this study, three fixed doses of TRN0011 (5 mg, 10 mg, and 15 mg) were selected in the trial and compared with the current standard of care for passive tetanus immunoprophylaxis, HTIG 250 IU, as well as placebo.

In this study, the mean serum anti-tetanus toxin neutralizing antibody ΔTiter at 6 hours post-dose reached 0.0113, 0.0342, and 0.0727 IU/mL for the TRN0011 5 mg, 10 mg, and 15 mg groups, respectively, thereby achieving the minimum protective level of 0.01 IU/mL. At this time point, the proportions of subjects with ΔTiter ≥0.01 IU/mL were 55.7%, 80.3%, and 96.6%, respectively. At the same time point, the mean ΔTiter following administration of HTIG 250 IU was 0.0050 IU/mL, and only 20.3% of subjects achieved ΔTiter ≥0.01 IU/mL, which is insufficient to provide ideal tetanus protection to the population.

Considering that the incubation period of tetanus is most commonly 3-21 days after injury and that clinical reports indicate that only approximately 10% of injured patients develop tetanus symptoms within less than 2 days, the primary endpoint of this study was selected at 24 hours post-dose for the evaluation of tetanus toxin neutralizing antibody titers.

At the primary endpoint, 24 hours post-dose, the mean neutralizing antibody titer ΔTiter in the TRN0011 5 mg, 10 mg, and 15 mg groups increased to 0.0450, 0.1095, and 0.2094 IU/mL, respectively, and the proportion of volunteers with ΔTiter ≥ 0.01 IU/mL further increased to 91.8%, 100%, and 100%, respectively. This indicates that all three doses selected in this study provided tetanus protection in a broad population at 24 hours post-dose.

Further evaluation of relevant secondary endpoints showed that TRN0011 10 mg and 15 mg provided protection in a high proportion of the study population as early as 12 hours post-dose. At this time point, the mean ΔTiter values for the two dosing groups were 0.0711 and 0.1476 IU/mL, respectively, and the proportion of volunteers with ΔTiter ≥ 0.01 IU/mL was 91.8% and 100%, respectively. The data further demonstrated that the protective effect of TRN0011 10 mg and 15 mg was sustained through the end of the study. At Day 106 post-dose, the mean ΔTiter values remained at 0.0241 and 0.0444 IU/mL, respectively, with the proportions of volunteers achieving ΔTiter ≥0.01 IU/mL maintained at 96.6% and 96.4%.

Data comparison between TRN0011 10 mg and 15 mg in terms of tetanus protection showed that, except for a higher proportion of protected subjects at 6 hours post-dose with the 15 mg dose, no additional apparently meaningful benefit was observed for the 15 mg dose compared with the 10 mg dose at other time points with respect to the proportion of protected subjects or the duration of protection. This study also found that, compared with HTIG 250 IU, administration of TRN0011 5 mg, 10 mg and 15 mg resulted in a faster and greater increase in serum anti-tetanus toxin neutralizing antibody titers. While 91.8% of volunteers in the TRN0011 10 mg group achieved ΔTiter ≥0.01 IU/mL at 12 hours post-dose, only 55.9% of volunteers in the HTIG group reached this protective level at the same time point, and it was not until 48 hours post-dose that the HTIG group achieved a comparable proportion (93.2%). In addition, at Day 60 post-dose, HTIG 250 IU was able to maintain ΔTiter ≥0.01 IU/mL in only 64.3% of volunteers, whereas the tetanus protective effect of TRN0011 10 mg and 15 mg persisted through the end of the study (Day 106 post-dose).

At all post-dose time points, the neutralizing antibody titers observed in the TRN0011 5 mg, 10 mg, and 15 mg groups were consistently higher than those in the HTIG 250 IU group.

The PK parameters observed in this study were generally consistent with those observed in those completed trials. Serum drug concentration increased with dose in an approximately linear manner. The time to reach peak serum drug concentration ranged from 6.0 to 13.0 days, and the half-life ranged from 25.1 to 26.8 days.

With respect to immunogenicity, only a very low proportion of volunteers (2.65%) developed TRN0011-related immunogenic reaction. The highest titer observed in ADA-positive samples was 6.14, and none of them exhibited neutralizing activity. Comparisons of PK parameters and summary of AE date indicated that immunogenicity had no apparent impact on individual PK or safety outcomes. This is consistent with the immunogenicity results of TRN0011 shown in previous studies .

In this study, the incidence of TEAEs in the TRN0011 groups was comparable to that observed in the HTIG and placebo groups, and all TEAEs were of mild or moderate severity. The most common TEAEs were blood triglycerides increased and blood uric acid increased. The incidence of these two types of TEAEs was similar in the TRN0011 group and the placebo group, at 10.6% vs 16.7% and 8.6% vs 6.7%, respectively.

In summary, this study further demonstrates that TRN0011 has a favorable safety and tolerability profile. Based on the anti-tetanus toxin neutralizing antibody titer analysis, TRN0011 provides better tetanus protection compared with HTIG.

The above description of specific embodiments of the present invention is not intended to limit the invention. Various modifications or variations may be made by those skilled in the art without departing from the spirit of the invention, and such modifications or variations shall fall within the scope of the appended claims.

## Claims

1. Use of an anti-tetanus toxin antibody in the preparation of a medicament for the prevention or treatment of tetanus;
the anti-tetanus toxin antibody comprises: heavy chain CDR1, heavy chain CDR2 and heavy chain CDR3 respectively comprising the amino acid sequences set forth in SEQ ID NO. 1, SEQ ID NO. 2 and SEQ ID NO. 3; and light chain CDR1, light chain CDR2 and light chain CDR3 respectively comprising the amino acid sequences set forth in SEQ ID NO. 6, SEQ ID NO. 7 and SEQ ID NO. 8.

2. The use according to claim 1, **characterized in that** the anti-tetanus toxin antibody comprises: a heavy chain variable region comprising the amino acid sequence set forth in SEQ ID NO. 4; and a light chain variable region comprising the amino acid sequence set forth in SEQ ID NO. 9;
preferably, the anti-tetanus toxin antibody comprises all or part of a heavy chain constant region and/or a light chain constant region;
more preferably , the anti-tetanus toxin antibody is a monoclonal antibody comprising two heavy chains and two light chains;
further preferably, the anti-tetanus toxin antibody comprises: a heavy chain comprising the amino acid sequence set forth in SEQ ID NO. 5; and a light chain comprising the amino acid sequence set forth in SEQ ID NO. 10.

3. The use according to claim 1 or 2, **characterized in that**, the medicament is used in a subject who has been infected with or has not yet been infected with *Clostridium tetani*, and the subject is a mammal; preferably, the subject is a primate, more preferably a human, such as an adult, and even more preferably an adult aged 18 years or older ;
and/or,
tetanus is prevented or treated by a single administration of the medicament to the subject; preferably, the medicament is an intramuscular injection preparation for administration of the medicament.

4. The use according to any one of claims 1 to 3, **characterized in that** the antibody dose achieved by administering the medicament ranges from 0.6 mg to 15 mg, for example 0.6 mg, 1.8 mg, 2.1 mg, 5 mg, 6 mg, 10 mg or 15 mg; preferably, the antibody dose achieved ranges from 5 mg to 15 mg; more preferably, the antibody dose achieved is 10 mg;
and/or,
based on the body weight of the subject, the antibody dose achieved by administering the medicament ranges from 10 µg/kg to 250 µg/kg, for example 10 µg/kg, 30 µg/kg, 35 µg/kg, 83 µg/kg, 100 µg/kg, 167 µg/kg or 250 µg/kg; preferably, the achieved antibody dose ranges from 83 µg/kg to 250 µg/kg; more preferably, the achieved antibody dose is 167 µg/kg.

5. The use according to any one of claims 1 to 4, **characterized in that** the medicament is used to achieve an anti-tetanus neutralizing antibody titer ≥ 0.01 IU/mL in the subject;
preferably, the anti-tetanus neutralizing antibody titer is detectable in the serum of the subject;
more preferably, the medicament is used to achieve an anti-tetanus neutralizing antibody titer ≥ 0.01 IU/mL in the subject within 48 h, preferably 24 h, more preferably 12 h, further preferably 6 h, or even less after dosing;
and/or,
the medicament is used to achieve an anti-tetanus neutralizing antibody titer lasting for more than 90 days, preferably lasting for more than 105 days, in the subject.

6. An anti-tetanus toxin antibody for use in a method comprising administering the antibody to a subject in need thereof to prevent or treat tetanus;
the anti-tetanus toxin antibody comprises: heavy chain CDR1, heavy chain CDR2 and heavy chain CDR3 respectively comprising the amino acid sequences set forth in SEQ ID NO. 1, SEQ ID NO. 2, and SEQ ID NO. 3; and
light chain CDR1, light chain CDR2 and light chain CDR3 respectively comprising the amino acid sequences set forth in SEQ ID NO. 6, SEQ ID NO. 7 and SEQ ID NO. 8.

7. The anti-tetanus toxin antibody according to claim 6, **characterized in that** the anti-tetanus toxin antibody comprises: a heavy chain variable region comprising the amino acid sequence set forth in SEQ ID NO. 4; and a light chain variable region comprising the amino acid sequence set forth in SEQ ID NO. 9;
preferably, the anti-tetanus toxin antibody comprises all or part of a heavy chain constant region and/or a light chain constant region;
more preferably, the anti-tetanus toxin antibody is a monoclonal antibody comprising two heavy chains and two light chains;
further preferably, the anti-tetanus toxin antibody comprises: a heavy chain comprising the amino acid sequence set forth in SEQ ID NO. 5; and a light chain comprising the amino acid sequence set forth in SEQ ID NO. 10.

8. The anti-tetanus toxin antibody according to claim 6 or 7, **characterized in that** the subject is a mammal that has been infected with or has not yet been infected with *Clostridium tetani*; preferably, the subject is a primate, more preferably a human, such as an adult, and even more preferably an adult aged 18 years or older;
and/or,
the method includes administering the anti-tetanus toxin antibody to the subject in a single dose to prevent or treat tetanus; preferably, the anti-tetanus toxin antibody is administered via intramuscular injection.

9. The anti-tetanus toxin antibody according to any one of claims 6 to 8, **characterized in that** the antibody dose administered in the method ranges from 0.6 mg to 15 mg, for example 0.6 mg, 1.8 mg, 2.1 mg, 5 mg, 6 mg, 10 mg or 15 mg; preferably, the antibody dose ranges from 5 mg to 15 mg; more preferably, the antibody dose is 10 mg;
and/or,
based on the body weight of the subject, the antibody dose administered in the method ranges from 10 µg/kg to 250 µg/kg, for example, 10 µg/kg, 30 µg/kg, 35 µg/kg, 83 µg/kg, 100 µg/kg, 167 µg/kg or 250 µg/kg; preferably, the antibody dose ranges from 83 µg/kg to 250 µg/kg; more preferably, the antibody dose is 167 µg/kg.

10. The anti-tetanus toxin antibody according to any one of claims 6 to 9, **characterized in that** the method achieves an anti-tetanus neutralizing antibody titer ≥ 0.01 IU/mL in the subject;
preferably, the anti-tetanus neutralizing antibody titer is detectable in the serum of the subject;
more preferably, the medicament is used to achieve an anti-tetanus neutralizing antibody titer ≥ 0.01 IU/mL in the subject within 48 h, preferably 24 h, more preferably 12 h, further preferably 6 h, or even less after dosing;
and/or,
the method achieves the anti-tetanus neutralizing antibody titer lasting for more than 90 days, preferably lasting for more than 105 days, in the subject.

11. A method for preventing or treating tetanus, the method comprising administering an anti-tetanus toxin antibody to a subject in need thereof;
the anti-tetanus toxin antibody comprises: heavy chain CDR1, heavy chain CDR2 and heavy chain CDR3 respectively comprising the amino acid sequences set forth in SEQ ID NO. 1, SEQ ID NO. 2 and SEQ ID NO. 3; and light chain CDR1, light chain CDR2 and light chain CDR3 respectively comprising the amino acid sequences set forth in SEQ ID NO. 6, SEQ ID NO. 7 and SEQ ID NO. 8.

12. The method according to claim 11, wherein the anti-tetanus toxin antibody comprises: a heavy chain variable region comprising the amino acid sequence set forth in SEQ ID NO. 4; and a light chain variable region comprising the amino acid sequence set forth in SEQ ID NO. 9;
preferably, the anti-tetanus toxin antibody comprises all or part of a heavy chain constant region and/or a light chain constant region;
more preferably, the anti-tetanus toxin antibody is a monoclonal antibody comprising two heavy chains and two light chains;
further preferably, the anti-tetanus toxin antibody comprises: a heavy chain comprising the amino acid sequence set forth in SEQ ID NO. 5; and a light chain comprising the amino acid sequence set forth in SEQ ID NO. 10.

13. The method according to claim 11 or 12, **characterized in that** the subject is a mammal that has been infected with or has not yet been infected with *Clostridium tetani*; preferably, the subject is a primate, more preferably a human, such as an adult, and even more preferably an adult aged 18 years or older;
and/or,
the method includes administering the anti-tetanus toxin antibody to the subject in a single dose to prevent or treat tetanus; preferably, the anti-tetanus toxin antibody is administered via intramuscular injection.

14. The method according to any one of claims 11 to 13, **characterized in that** the antibody dose administered in the method ranges from 0.6 mg to 15 mg, for example 0.6 mg, 1.8 mg, 2.1 mg, 5 mg, 6 mg, 10 mg or 15 mg; preferably, the antibody dose ranges from 5 mg to 15 mg; more preferably, the antibody dose is 10 mg;
and/or,
based on the body weight of the subject, the antibody dose administered in the method ranges from 10 µg/kg to 250 µg/kg, for example, 10 µg/kg, 30 µg/kg, 35 µg/kg, 83 µg/kg, 100 µg/kg, 167 µg/kg or 250 µg/kg; preferably, the antibody dose ranges from 83 µg/kg to 250 µg/kg; more preferably, the antibody dose is 167 µg/kg.

15. The method according to any one of claims 11 to 14, **characterized in that** the method achieves an anti-tetanus neutralizing antibody titer ≥ 0.01 IU/mL in the subject;
preferably, the anti-tetanus neutralizing antibody titer is detectable in the serum of the subject;
more preferably, the medicament is used to achieve an anti-tetanus neutralizing antibody titer ≥ 0.01 IU/mL in the subject within 48 h, preferably 24 h, more preferably 12 h, further preferably 6 h, or even less after dosing;
and/or,
the method achieves the anti-tetanus neutralizing antibody titer lasting for more than 90 days, preferably lasting for more than 105 days, in the subject.
